Europäisches Patentamt

European Patent Office

Office européen des brevets

(19)

(11) Veröffentlichungsnummer: **0 330 977**

**A2**

# (12) EUROPÄISCHE PATENTANMELDUNG

(21) Anmeldenummer: **89103030.6**

(22) Anmeldetag: **21.02.89**

(51) Int. Cl.⁴: **C12N 15/00**

(30) Priorität: **02.03.88 DE 3806617**

(43) Veröffentlichungstag der Anmeldung:
**06.09.89 Patentblatt 89/36**

(84) Benannte Vertragsstaaten:
**AT BE CH DE ES FR GB GR IT LI LU NL SE**

(71) Anmelder: **BEHRINGWERKE
Aktiengesellschaft
Postfach 1140
D-3550 Marburg 1(DE)**

(72) Erfinder: **Zettlmeissl, Gerd, Dr.
Am Hofacker 15
D-3551 Lahntal(DE)**
Erfinder: **Langner, Klaus-Dieter, Dr.
Lindenweg 14
D-3550 Marburg(DE)**
Erfinder: **Wirth, Manfred, Dr.
Marktstrasse 1
D-3340 Wolfenbüttel(DE)**
Erfinder: **Hauser, Hansjörg, Dr.
Georg-Westermann-Allee 29
D-3300 Braunschweig(DE)**

(74) Vertreter: **Meyer-Dulheuer, Karl-Hermann, Dr.
et al
HOECHST Aktiengesellschaft Postfach 3540
D-6121 Wiesbaden(DE)**

(54) **Erzeugung hochexprimierender rekombinanter eukaryotischer Zellen.**

(57) Es wird eine Methode beschrieben, wobei durch Kotransfektion von mindestens zwei selektierbaren Genen und anschließende Selektion ein oder mehrere nicht selektierbare Gene zu einer wesentlich erhöhten Expression gebracht werden können. In anschließenden Schritten kann zur weiteren Expressionssteigerung auf jeden Genmarker einzeln selektiert werden.

EP 0 330 977 A2

## Erzeugung hochexprimierender rekombinanter eukaryotischer Zellen

Die Transfektion von DNA ist eine weitverbreitete Methode zur Übertragung fremder Gene auf animale Zellen in Kultur. Mit Hilfe dieser Technik konnten in den letzten Jahren zum einen eine Reihe regulatorischer DNA-Regionen unterschiedlicher Gene identifiziert und in ihrer Funktion analysiert werden und zum anderen pharmazeutisch interessante Proteine in ihrer nativen oder weitgehend nativen Form dargestellt werden.

Für den Gentransfer können heute eine Reihe verschiedener Techniken eingesetzt werden. Je nach der verwendeten Technik integrieren die transfizierten Gene als Einzelkopie oder als assoziierte Konkatemere in einem statistischen Prozeß in das Chromosom der Wirtszelle. Die Expressionsrate für das (die) transfizierte-(n) Gen(e) hängt hierbei von einer Reihe von Faktoren ab: z. B. von der Kopienzahl, dem Integrationsort, der Stärke des verwendeten Promotors bzw. Enhancers und der Stabilität der mRNA.

Bei dem gegebenen Stand der Technik stellt der zielgerichtete Einbau der fremden DNA in Bereiche des Chromosoms mit hoher transkriptioneller Aktivität ein noch weitgehend ungelöstes Problem dar. Die Kopienzahl jedoch kann manipuliert werden. Da ein Zusammenhang zwischen der Kopienzahl und den Expressionshöhen besteht, sind Systeme, die die Möglichkeit der Genamplifikation bieten, von Interesse, um ein Gen in hohen Ausbeuten zu exprimieren. In den letzten Jahren wurde eine Reihe von amplifizierbaren eukaryotischen Genen beschrieben. Unter letzteren ist das Dihydrofolatreduktasegen (DHFR), das in Zellen als Antwort auf ansteigende Konzentrationen des Inhibitors Methotrexat (MTX) amplifiziert wird, am besten charakterisiert. Eine "Chinese Hamster Ovary"-Zellinie (CHO), die kein funktionelles endogenes DHFR-Gen mehr besitzt (Urlaub und Chasin, 1980, Proc. Natl. Acad. Sci. USA 77, 4216-4220), eignet sich hierbei besonders für die Amplifikation eines exogenen DHFR-Gens und daran gekoppelter Gene (Kaufman und Sharp, 1982, J. Mol. Biol. 159, 601-621).

Jedoch ist mit dieser Methode das "Screening" auf gut produzierende Zellinien sehr arbeits- und zeitaufwendig, da für ausreichende Amplifikation und Expression in der Regel 4 bis 8 Monate benötigt werden. Außerdem ist die Amplifikation über MTX bisher auf das CHO-system oder äquivalente Zellinien beschränkt,da sie in anderen nicht DHFR-defizienten Zellen häufig von unspezifischer Resistenz begleitet wird. Hinzu kommt, daß eine kompetierende Amplifikation des endogenen DHFR-Gens nicht ausgeschlossen werden kann, was den gewünschten Amplifikationseffekt gegebenenfalls drosselt.

Die vorliegende Erfindung ist eine Methode zum direkten und schnellen "Screening" auf hochproduzierende Zellinien beliebigen Ursprungs bzw. Wildtyp-Zellinien. Bei dieser Methode werden zunächst mit dem (den) nicht selektierbaren Gen(en) von Interesse mindestens zwei selektierbare Gene in die entsprechende Zelle bzw. Wildtypzelle kotransfiziert. Es ist bekannt, daß Kotransfektion eine sehr effiziente Methode ist, um getrennte Gene gleichzeitig in animale Zellen einzuführen. Hier soll unter Kotransfektion auch ganz allgemein die Transfektion von Vektoren oder DNA-Strukturen verstanden werden. Kotransfizierte DNA-Fragmente werden in der Regel in direkter Nachbarschaft zueinander in das Wirtschromosom integriert und werden in vielen Fällen in hoher Korrelation exprimiert. Anschließend wird in einem ersten Schritt durch gleichzeitige oder kurz aufeinanderfolgende Zugabe der entsprechenden (Hemm-) Substanzen zum Medium auf Resistenz bezüglich aller Marker selektiert. Bevorzugt ist die gleichzeitige Zugabe bzw. Zugabe innerhalb einer Stunde, aber die Erfindung erstreckt sich auch auf die Zugabe in Abständen von 1 Stunde bis 120 Stunden. Hierbei erhält man Zellinien mit deutlich erhöhten Expressionsraten für das nicht-selektierbare Gen verglichen mit Zellen, die nur auf ein Markergen selektioniert worden waren. Dabei ergibt sich nicht nur ein additiver, sondern ein multiplikativer Effekt für die erzielte Expression. Mit Hilfe der Verwendung von zwei oder mehr selektierbaren Markern werden bei diesem System Schwierigkeiten vermieden, die auf unspezifischen Resistenzen beruhen. Die genannten "Markergene" können dabei mit dem nicht selektierbaren Gen von Interesse zusammen auf einem Vektor liegen oder auf mehrere Vektoren verteilt sein.

Weiterhin ist es nun möglich, durch Erhöhung der Konzentration eines (mehrerer) der selektierenden Substanzen im Kulturmedium unter Konstanthaltung des (der) anderen auf Zellen mit gesteigerten Kopienzahlen für alle transfizierten Gene und insbesondere mit hohen Expressionsraten für das nicht-selektierbare Gen zu selektionieren.

Der Vorteil dieser Methode liegt in ihrer breiten Anwendbarkeit auf beliebige Zellinien und der Vermeidung langwieriger Selektions- und Screeningprozeduren. Damit können z. B. Proteinmutanten in kurzer Zeit in den für Wirksamkeitsstudien benötigten Mengen gentechnisch erzeugt und anschließend ausgetestet werden.

Die Erfindung betrifft demnach das obengenannte Verfahren zur Erzeugung hochexprimierender rekombinanter Zellen sowie seine Verwendung zum Screening von Mutanten nicht selektierbarer Gene. Weitere

EP 0 330 977 A2

Ausgestaltungen der Erfindung sind in den nachfolgenden Beispielen und den Patentansprüchen aufgeführt.

**Beispiele**

1. Doppelselektion von "Baby Hamster Kidney" (BHK)-Zellen

Die Zahl der transfizierten Gene ist bei der oben beschriebenen Kotransfektionsmethode nicht der limitierende Faktor, so daß durchaus mehr als zwei Gene gleichzeitig in die Zelle eingebracht werden können. Am Beispiel der Kotransfektion einer Transkriptionseinheit für humanes Antithrombin III mit unterschiedlichen Selektionsmarkerkombinationen (Neomycin-Resistenz/DHFR; Neomycin-Resistenz/Puromycin-Resistenz) in BHK-Zellen sollen die vorteilhaften Eigenschaften des vorgestellten Doppelselektionssystems zur Expression eines heterologen Strukturgens von Interesse aufgezeigt werden.

1. a) Kotransfektion von Plasmid pSVATIII mit den Plasmiden pAG60/pAdDHFR (Neomycin-Resistenz/DHFRcDNA)

BHK 21-(ATCC CCL-10) Zellen wurden für dieses Experiment in Dulbecco's Modified Eagles (DME) Medium mit 10 % Neugeborenen-Kalbsserum (NCS) vermehrt.

Zur Transfektion wurden folgende zirkuläre Plasmid-DNAs mit der Kalziumphosphattechnik (Graham und van der Eb (1973) Virology 52, 456-467)) in einem Volumen von 0,5 ml kopräzipitiert: 2 $\mu$g pSVATIII (Zettlmeißl et al. (1987) Biotechnology 5, 720-725) 0,8 $\mu$g pAdDHFR (Kaufman und Sharp, 1982, a.a.O.) und 0,2 $\mu$g pAG60 (Colbère-Garapin et al. (1981) J. Mol. Biol. 150, 1-14). Das Kopräzipitat wurde mit 5 ml Medium auf 1,2 x $10^5$ Zellen in ein 25 cm²-Kulturgefäß gegeben und für 16 Stunden bei 37° C inkubiert. Nach weiteren 48 Stunden Inkubation der Zellen mit 5 ml DME/10 % NCS wurden die Zellen 1:3 in DME/10 % NCS Medium + 500 $\mu$g/ml G418 (Geneticin, Gibco) + 0 - 300 nM MTX umgesetzt. Nach 6 - 14 Tagen Selektionszeit (Mediumwechsel alle 3 - 4 Tage) wurden die resistenten Klone gezählt, abtrypsiniert und als Klonmischung (Mischklon, d. h. Pool der erhaltenen Klone) vermehrt.

Zur Bestimmung der AT III-Expressionsrate wurden 5 x $10^5$ Zellen eines solchen Mischklons in einem 25 cm²-Kulturgefäß mit 5 ml Medium angelegt. Nach 24 Stunden wurde für weitere 24 Stunden frisches Medium zugegeben.

Die Zellen wurden gezählt und der AT III-Gehalt wurde mit einem spezifischen Enzmye Linked Immunosorbent Assay (ELISA) bestimmt (vorgeschlagen in DPA P3624453.8).

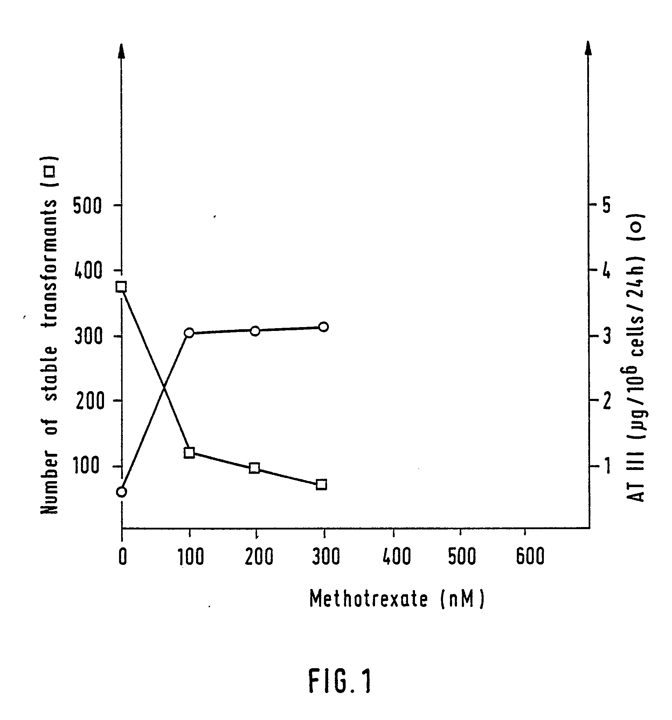

Fig. 1 zeigt, daß die Zahl der Klone pro Transfektion mit steigender MTX-Konzentration abnimmt, wogegen die Menge des sezernierten AT III bei Doppelselektion bis zu viermal höher liegt als bei einfacher Selektion mit G418.

Aus bei unterschiedlichen MTX-Konzentrationen doppelselektierten BHK-Klonen wurde die chromosomale DNA isoliert und im Dot-Blot-Verfahren (Maniatis et al. (1982) Molecular Cloning - A Laboratory Manual. Cold Spring Harbour, 158, 393-401) mit einem mittels Nick-Translation $^{32}$P-markierten Fragment aus der AT III cDNA hybridisiert. Die Ergebnisse zeigen, daß zunehmende Resistenz gegenüber MTX mit einer Erhöhung der mittleren AT III-Gen-Kopienzahl der Klonmischungen und der AT III-Expressionsraten korreliert.

1. b) Kotransfektion des Plasmids pAB3-1 (AT III) mit den Plasmiden pRMH140/pSV2dhfr (Neomycin-Resistenz/DHFRcDNA)

Dieses Beispiel soll zeigen, daß das in Beispiel 1a) vorgestellte System mit leicht verändertem Protokoll auch mit Vektoren, die andere Transkriptionsregulationssequenzen für die jeweiligen Gene (AT III, Neomycin-Resistenz, DHFR) tragen, durchgeführt werden kann.

BHK-Zellen wurden für diese Experimente in DME-Medium, das 10 % Foetales Kalbsserum (FKS) enthielt (Kulturmedium), vermehrt. Zur Transfektion wurden folgende zirkuläre Plasmid-DNAs mit der Kalziumphosphattechnik (s. o.) in einem Volumen von 1 ml kopräzipitiert: 20 $\mu$g pAB3-1, 5 $\mu$g pSV2dhfr (Lee et al. (1981) Nature 294, 228-232) und 5 $\mu$g pRMH140 (Hudziak et al. (1982) Cell 31, 137-146). pAB3-1 setzt sich aus dem Plasmid pSVATIII zusammen, in dessen EcoRI-Schnittstelle die "Enhancer-Region" von humanem Cytomegalovirus, Position-147 bis -598 (Boshart et al. (1985) Cell 41, 521-530) hineinligiert

3

wurde. Das Kopräzipitat wurde direkt für 30 Minuten bei 37°C auf 5 x 10$^5$ Zellen in einem 25 cm²-Kulturgefäß gegeben. Nach Zugabe von 5 ml Kulturmedium wurden die Zellen weitere 5 - 6 Stunden bei 37°C inkubiert. Danach wurden die Zellen 3 Minuten lang bei 37°C mit 15 % (v/v) Glycerin in Kulturmedium behandelt. Nach zweimaligem Waschen mit Kulturmedium wurden die Zellen 72 Stunden lang bei 37°C in Kulturmedium inkubiert. Zur Selektion wurden die Zellen anschließend 1:3 bis 1:4 in Kulturmedium, das 400 µg/ml G418 mit und ohne 1 µM MTX enthielt, umgesetzt.

Nach 10 - 15 Tagen Selektionszeit (Mediumwechsel alle 3 -4 Tage) wurden die resistenten Klone gezählt, abtrypsiniert und als Klonmischung vermehrt. Die Bestimmung der AT III-Expressionsrate erfolgte wie in Beispiel 1a) beschrieben.

Tabelle 1 zeigt, daß bei Doppelselektion die Anzahl der auftretenden Klone pro Transfektion etwa um Faktor fünf niedriger liegt als bei Selektion mit G418 allein. Weiterhin ist ersichtlich, daß die AT III-Expressionsraten nach Doppelselektion um etwa Faktor sechs höher sind.

Tabelle 1

| G418 [µg/ml] | MTX [µM] | Kolonien/ # Transfektion | ATIII-Expression # [µg/10$^6$ Zellen/24 h] |
|---|---|---|---|
| 400 | 0 | 300 ± 30 | 1 ± 0,2 |
| 400 | 1 | 60 ± 15 | 6 ± 0,3 |

# Mittelwert aus drei unabhängigen Transfektionsexperimenten

Ein doppelselektionierter Mischklon (3MK1; s. Tabelle 2) wurde stufenweise höheren MTX-Konzentrationen im Medium ausgesetzt (1 → 10 µM). Hierbei konnte eine Verdopplung der AT III-Expressionsrate erzielt werden. Die nach einer quantitativen Southernblotmethode (Zettlmeißl et al., a.a.O.) bestimmte mittlere Kopienzahl für AT III nimmt bei dieser sekundären MTX-Selektion ebenfalls zu (Tabelle 2).

Tabelle 2

| G418 [µg/ml] | MTX [µM] | ATIII Genkopien/Zelle (3MK1) | ATIII-Expression [µg/10$^6$ Zellen/24 h] |
|---|---|---|---|
| 400 | 1 | 40 | 6 ± 0,2 |
| 400 | 10 | 150 | 12 ± 0,4 |

1. c) Kotransfektion von Plasmid pSVATIII mit den Plasmiden pAG60/pSV2PAC (Neomycin-Resistenz/Puromycin-Resistenz)

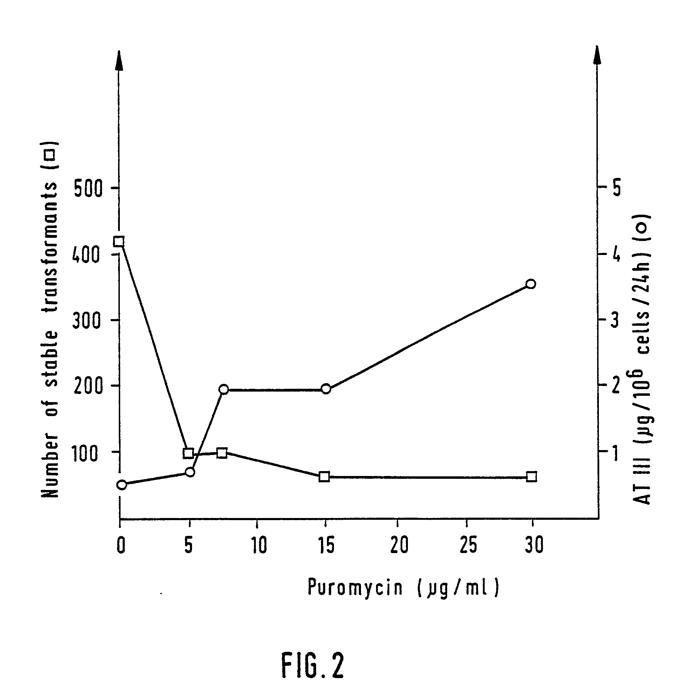

BHK-Zellen wurden wie in Beispiel 1a) beschrieben kotransfiziert, wobei an Stelle des Plasmids pAdDHFR 0,8 µg des Plasmids pSV2PAC (Vara et al. (1986) Nucl. Acids Res. 11, 4617-4624) eingesetzt wurde. (pSV2PAC kodiert für eine N-Acetyltransferase aus Streptomyces alboniger und verleiht bei Expression in animalen Zellen Resistenz gegenüber dem Antibiotikum Puromycin.) Die transfizierten Zellen wurden in DME/10 % NCS-Medium + 500 µg/ml G418 + 0 - 30 µg/ml Puromycin selektiert, als Mischklone vermehrt und wie oben beschrieben auf AT III-Expression getestet. Auch in diesem Experiment nimmt die Anzahl der erhaltenen Klone pro Transfektion mit steigender Puromycinkonzentration ab. Die AT III-Expressionsraten hingegen sind bei doppelselektierten Zellen um Faktor vier bis sechs höher als bei Zellen, die nur mit G418 selektiert worden waren (Fig. 2).

2. Doppelselektion in anderen Zellsystemen

In den folgenden Beispielen soll gezeigt werden, daß das Prinzip der Doppelselektion auch auf andere animale Zellen in Kultur angewandt werden kann.

4

2. a) Kotransfektion von Plasmid pSVtss+ (enthält cDNA für humanes Interferon-β) mit den Plasmiden pAG60/pSV2PAC (Neomycin-Resistenz/Puromycin-Resistenz) in CHO und Maus L TK⁻ -Zellen.

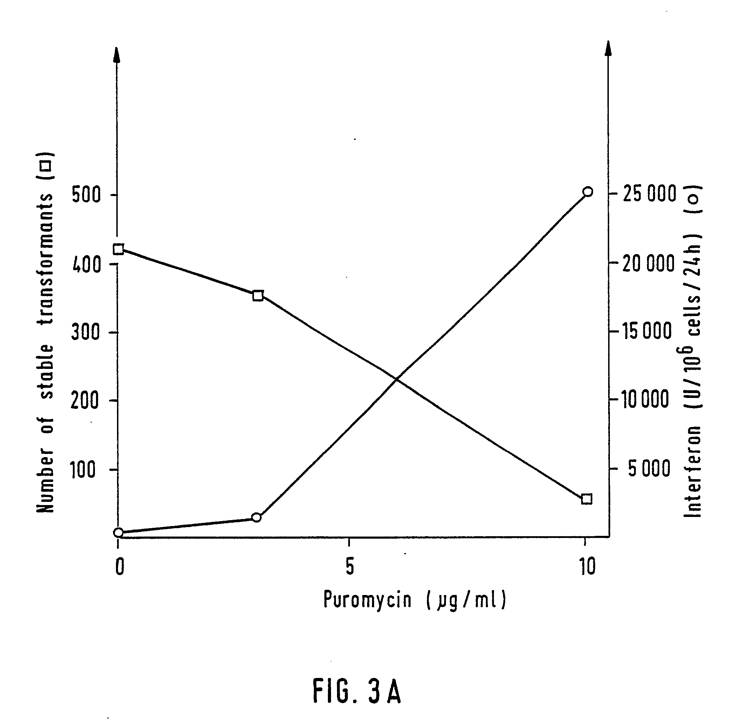

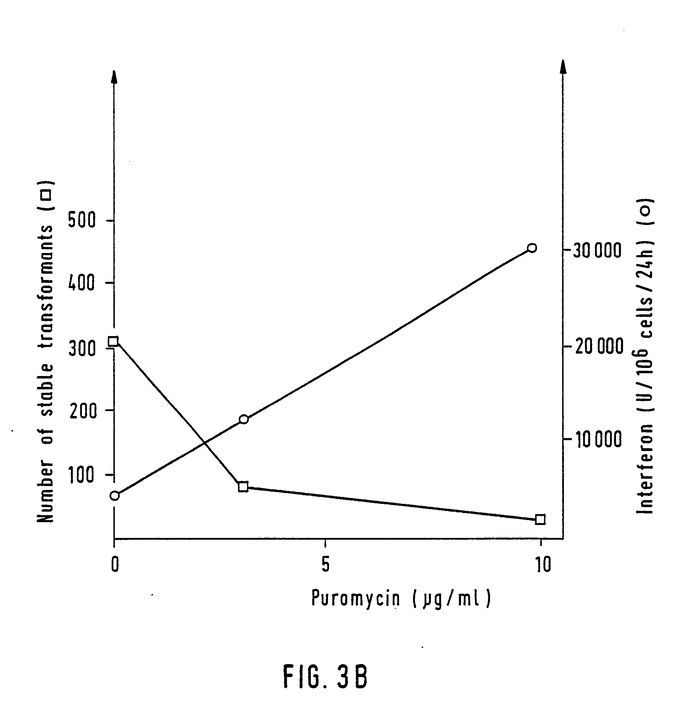

CHO dhfr⁻ (Urlaub und Chasin (1980) a.a.O.) und L TK⁻-(ATCC CCL1.3)-Zellen wurden wie in Beispiel 1a) und 1c) beschrieben mit folgenden zirkulären Plasmid-DNAs kotransfiziert: 2 μg pSVtss+ (Reiser und Hauser (1987) Drug Res., 37, 482-485), 0,2 μg pAG60 und 0,8 μg pSVPAC. Nach Selektion in DME/10 % NCS + G418 (360 μg/ml für CHO-Zellen; 700 μg/ml für L TK⁻ -Zellen) + Puromycin (0 - 10 μg/ml) wurde die Zahl der stabilen Transfektanten gezählt, die Zellen als Mischklone vermehrt und die Interferon-β-Expressionsraten über einen antiviralen Test (Finter (1969) J. Gen. Virol. 5, 419-427) bestimmt. Hierzu wurden konfluente Zellen 5 Stunden lang mit poly(I):poly(C) behandelt. Für den Test wurden 15 Stunden Überstände gesammelt (Dinter und Hauser (1987) EMBO J. 6, 599-604). CHO-Mischklone, die mit 10 μg/ml Puromycin und G418 doppelselektiert worden waren, exprimierten 20-fach mehr Interferon-β als solche, die nur einer G418-Selektion unterlagen (Fig. 3A). L TK⁻ -Mischklone, die mit 10 μg/ml Puromycin und G418 doppelselektiert worden waren, exprimierten etwa 6-fach mehr Interferon-β als solche, die nur einer G418-Selektion unterlagen (Fig. 3 B).

2. b) Kotransfektion von Plasmid pAB3788 (enthält cDNA für humanen Faktor VIII:C) mit den Plasmiden pRMH140/PSV2dhfr in HE7-zellen (Hamster)

Zur Konstruktion des Plasmids 3788 wurde eine cDNA, die für eine Deletionsmutante von humanem Faktor VIII:C kodiert (Deletion der Aminosäuren 741 bis 1689 wie in der deutschen Patentanmeldung P 37 20 246.4 vorgeschlagen) und von XhoI-Stellen flankiert ist, in den Vektor pAB3-1 (siehe Beispiel 1b) kloniert. Hierzu wurde pAB3-1 mit SalI/XbaI geschnitten, die überhängenden 5'Enden mit dem Klenow-Fragment der E. coli DNA-Polymerase I aufgefüllt und als Vektorfragment (3.3 kb) gereinigt. Nachdem die überhängenden 5'-Enden der XhoI-Stellen des gereinigten Faktor VIII:C-DNA-Fragments ebenfalls aufgefüllt worden waren, wurde durch Ligierung der Vektor pAB3788 erhalten. 20 μg des Plasmids pAB3788 wurden wie in Beispiel 1b) beschrieben mit den Plasmiden pRMH140 und pSV2dhfr in HE7-Zellen (Cook, J. L. und Lewin, A. M. (1979) Cancer Res. 39, 1455 - 1461) kotransfiziert. Nach im Beispiel 1b beschriebener Selektion wurden ca. 300 ± 30 Klone pro Transfektionsansatz erhalten. Als Mischklon vermehrte Transfektanden exprimierten 0,7 ± 0,1 Einheiten (U) Faktor VIII:C pro 10⁶ Zellen pro 24 h. Doppelt-selektionierte Zellen exprimierten damit um etwa den Faktor 3 bis 6 mehr der Faktor VIII:C-Mutante als in unabhängigen Experimenten mit G418 selektionierte Mischklone. Die Faktor VIII:C-Aktivität wurde mittels des COA-Tests (Kabi Vitrum, Uppsala, Schweden) bestimmt.

3. Doppelselektion als Methode zum Erreichen hoher Titer rekombinanter Retroviruspartikel

Zur Konstruktion des retroviralen Expressionvektors pM5AT III wurde ein 1,4 kb BglII/BamHI-Fragment (AT III cDNA) in die singuläre BamHI-Stelle von pM5neo kloniert, welche von einer Spleiß-Donorstelle bzw. Spleiß-Akzeptorstelle des myeloproliferativen Sarcomavirus flankiert ist. Das ATIII-Fragment entstand durch Anheften von BglII Linkern an das 1,6 kb SalI/EcoRI-Fragment aus dem Vektor pSVAT III (s.o.), gefolgt von einer BamHI/BGIII-Spaltung. pM5neo enthält neben einem pBR Rückgrat u.a. die o.g. Spleißstellen und das Neomycin-Resistenzgen sowie die LTRs (long terminal repeats) des myeloproliferativen Sarkomavirus (Ostertag et al. (1980) J. Virol. 33, 573-582; Stacey et al. (1984) J. Virol. 50, 725-732).

Die Helfer-Zellinie Psi2 (Mann et al. (1983) Cell 33, 153-159) wurde in DME-Medium mit 10% FKS vermehrt. Zur Transfektion wurde eine Mischung aus zirkulärer Plasmid-DNA (5 μg pM5AT III und 2 μg pSV2PAC) und 8 μg gescherter DNA aus Maus L-Zellen in einem Volumen von 0,5 ml mit Kalziumphosphat kopräzipitiert und nach 30-minütiger Inkubation bei Raumtemperatur zu 2 x 10⁵ Psi2-Zellen in 5 ml Kulturmedium gegeben. Nach etwa 12stündiger Inkubation bei 37° C wurde das Medium gewechselt und wiederum 24 h später durch Selektionsmedium (Einzelselektion mit 1 mg/ml G418, Doppelselektion mit 1 mg/ml G418 und 2 bzw. 5 μg/ml Puromycin in DME-Medium plus 10% FKS) ersetzt. Die entstehenden Klone wurden nach 10-14 Tagen gezählt und gepoolt.

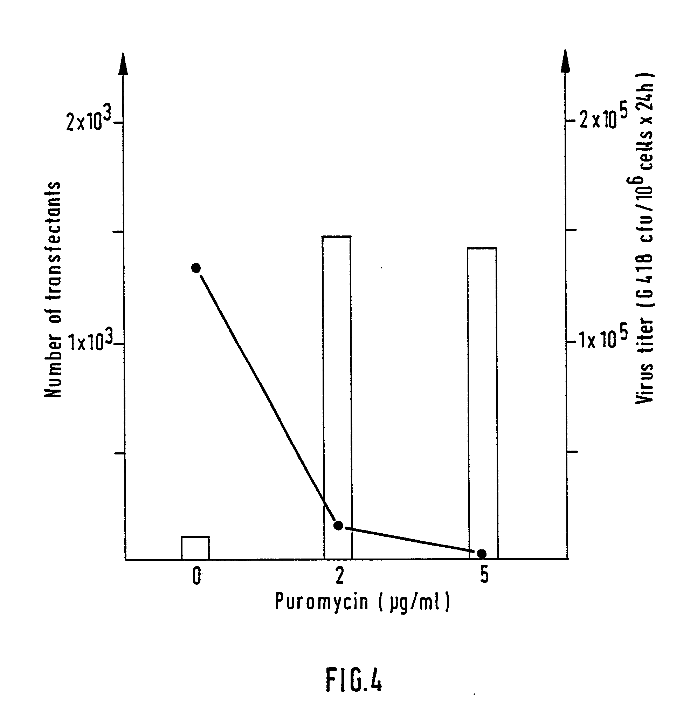

Zur Bestimmung des Virustiters wurden 5 x 10⁵ Psi2-Zellen ausgesät und das Medium nach 24 Stunden gewechselt. 24 Stunden später wurde Überstand, der rekombinante Retroviruspartikel enthält, oder entsprechende Verdünnungen zur Infektion von HIH 3T3-Zellen (ATCC CRL 1658; 1000-3000 Zellen pro 24-Loch Platte) verwendet. Die Infektion erfolgte unter Zugabe von 8 μg/ml Polybren über ca. 12 Stunden. Anschließend wurde das Medium gewechselt und Nach 24 Stunden durch Selektionsmedium ersetzt (1 mg ml G418). Die entstehenden Klone wurden 10 Tage nach dem Wechsel auf Selektionsmedium angefärbt und gezählt. Klonmischungen, die aus der Doppelselektion hervorgehen, erzielen etwa 10-fach höhere virale Titer als einzelselektierte Klonmischungen (Fig. 4)

Legende zu den Fig. 1 - 3

Die Anzahl der stabilen Transformanten ist auf der Ordinate links aufgetragen und durch offene Kästchen dargestellt. Die Menge des Genprodukts von Interesse (AT III-oder Interferon) ist auf der Ordinate rechts aufgetragen und durch offene Kreise dargestellt. Auf der Abszisse ist die Konzentration der jeweiligen variierten Inhibitoren (Methotrexat oder Puromycin) aufgetragen.

## Ansprüche

1. Verfahren zur Expression von nicht selektierbaren Genen in eukaryotischen Zellen, dadurch gekennzeichnet, daß zwei oder mehr Selektionsmarkergene mit den nicht selektierbaren Genen transfiziert werden, wobei sich die Selektionsmarkergene und die nicht selektierbaren Gene auf einem oder mehreren Vektoren oder DNA-Strukturen befinden und anschließend auf alle transfizierten Selektionsmarker selektiert wird.

2. Verfahren nach Anspruch 1, dadurch gekennzeichnet, daß gleichzeitig oder innerhalb einer Stunde auf alle transfizierten Selektionsmarker selektiert wird.

3. Verfahren nach Anspruch 1, dadurch gekennzeichnet, daß in Abständen von 1 Stunde bis 120 Stunden, vorzugsweise 2 bis 72 Stunden, auf die transfizierten Selektionsmarker nacheinander selektiert wird.

4. Verfahren nach Anspruch 1, 2 oder 3, dadurch gekennzeichnet, daß ein einzelnes, nicht selektierbares Gen kotransfiziert wird.

5. Verfahren nach Anspruch 1, 2, 3 oder 4, dadurch gekennzeichnet, daß in nachfolgenden Selektionsschritten die Konzentration einer oder mehrerer Hemmsubstanzen, bevorzugt einer Hemmsubstanz, um den Faktor 2 bis 100, vorzugsweise um den Faktor 8 bis 12, erhöht wird.

6. Verfahren nach Anspruch 1, 2, 3, 4 oder 5, dadurch gekennzeichnet, daß das nicht selektierbare Gen für AT III, F VIII : C, F XIII a, t-PA, EPO, G-CSF, GM-CSF, PAI, Protein C oder IL-3 kodiert.

7. Verfahren nach Anspruch 1, 2, 3, 4 oder 5 zum Screening von Mutanten nicht selektierbarer Gene.

FIG. 1

FIG.2

FIG. 3A

FIG. 3 B

FIG.4